# EUROPÄISCHE PATENTSCHRIFT

(11) **EP 1 001 811 B1**
(45) Veröffentlichungstag und Bekanntmachung des Hinweises auf die Patenterteilung: **11.09.2002**
(21) Anmeldenummer: 98939520.7
(22) Anmeldetag: 19.06.1998
(51) Int. Cl.: A61K 41/00, A61P 9/10, A61P 35/00

(54) **NANOSKALIGE TEILCHEN MIT EINEM VON MINDESTENS ZWEI SCHALEN UMGEBENEN EISENOXID-HALTIGEN KERN**
NANOSCALE PARTICLES HAVING AN IRON OXIDE-CONTAINING CORE ENVELOPED BY AT LEAST TWO SHELLS
PARTICULES DE L'ORDRE DE GRANDEUR DU NANOMETRE AVEC UN NOYAU CONTENANT DE L'OXYDE DE FER ET ENTOURE D'AU MOINS DEUX ENVELOPPES

(30) Priorität: 20.06.1997 DE 19726282
(43) Veröffentlichungstag der Anmeldung: 24.05.2000
(73) Patentinhaber: Institut Für Neue Materialien gem. GmbH, 66123 Saarbrücken (DE)
(72) Erfinder: LESNIAK, Christoph, D-87634 Obergünzburg (DE); SCHIESTEL, Thomas, D-66121 Saarbrücken (DE); SCHMIDT, Helmut, D-66130 Saarbrücken (DE); JORDAN, Andreas, D-14167 Berlin (DE)
(74) Vertreter: Barz, Peter, Dr.
(86) Internationale Anmeldenummer: EP9803761
(87) Internationale Veröffentlichungsnummer: WO98058673

(56) Entgegenhaltungen:
- EP-A- 0 472 990
- EP-A- 0 667 148
- WO-A-88/00060
- WO-A-90/01295
- WO-A-93/24076
- DE-A- 19 614 136
- HERMENTIN P ET AL: "HINGE-THIOL COUPLING OF MONOCLONAL ANTIBODY TO SILANIZED IRON OXIDE PARTICLES AND EVALUATION OF MAGNETIC CELL DEPLETION" BIOCONJUGATE CHEMISTRY, Bd. 1, Nr. 6, 1. November 1990, Seiten 411-418, XP000174781
- JUNG C W ET AL: "Physical and chemical properties of superparamagnetic iron oxide MR contrast agents: ferumoxides, ferumoxtran, ferumoxsil." MAGNETIC RESONANCE IMAGING, (1995) 13 (5) 661-74. JOURNAL CODE: MAK. ISSN: 0730-725X., XP002085566 United States

## Beschreibung

Die vorliegende Erfindung betrifft nanoskalige Teilchen mit einem Eisenoxid-haltigen ferri-, ferro- oder (vorzugsweise) superparamagnetischen Kern und mindestens zwei diesen Kern umgebenden Schalen. Diese Teilchen können für medizinische Zwecke eingesetzt werden, insbesondere für die Tumortherapie durch Hyperthermie.

In WO 90/01295 werden für die MR-Diagnostik geeignete Kontrastmittel aus einem Eisenoxid-haltigen Kern beschrieben, der z.B. mit Aminosilanen oder Dextran umgeben ist. Die Aufnahme dieser Teilchen in Zellen erfolgt durch rezeptorvermittelte Endocytose (RME). Die Teilchen weisen hierfür spezifische Liganden auf, über die eine Bindung an Rezeptoren von Zellen ermöglicht wird.

Bioconjugate Chemistry, Bd. 1, Nr. 6, S. 411-418, betrifft Eisenoxid-haltige Teilchen mit einer Abmessung von 0,5 bis 1,5 µm, die von Aminosilanen ummantelt sind. Über diese Aminogruppen werden monoklonale Antikörper kovalent verknüpft. Über diese Antikörper können die Teilchen an Rezeptoren von Zelloberflächen gebunden werden.

WO 88/00060 offenbart ebenfalls Eisenoxid-haltige Kerne, die eine Umhüllung mit Dextran oder Aminosilanen aufweisen können. An diese Umhüllung können biologische Moleküle angebracht werden, die zur Bindung an das gewünschte Target dienen. Die Teilchen können für MR-Kontrastmittel verwendet werden.

Es wird allgemein angenommen, daß transformierte Zellen (Krebszellen) der meisten Tumoren im Vergleich zu ihren benignen Gegenstücken eine höhere Phagozytoseaktivität aufweisen. Als Gründe hierfür werden die Invasionstätigkeit in benachbarte Gewebe und die damit verbundene Exozytose lytischer Enzyme sowie die höhere Stoffwechselaktivität dieser transformierten Zellen angenommen. Im Streben nach einer schnellen Zellvermehrung entdifferenzieren sich die Krebszellen und verlieren dadurch einen Teil ihrer Spezifität hinsichtlich normaler Signaltransduktionswege und allgemein hinsichtlich transmembraner Vorgänge. In jüngster Zeit wurden solche Veränderungen z.B. am Verlust/an der Veränderung der wichtigen Zelladhäsionsproteine und Glycoproteine der Zelloberfläche erkannt; der bzw. die inzwischen als eine der Voraussetzungen für das ungehemmte Wachstum maligner Zellen angesehen wird. So weiß man, daß Krebszellen ähnlich den Makrophagen Fragmente normaler Zellen oder anderer Zelltrümmer in sich aufnehmen und in Lysosomen endogen in verwertbare Näbrstoffe umsetzen können. Die Signalerkennung, mit der der Endozytoseprozeß bei Tumorzellen ausgelöst wird, ist im Prinzip noch unklar. Aus in vivo-Untersuchungen intraläsional applizierter Partikelsuspensionen geht jedoch hervor, daß die Verteilung im Gewebe und die Aufnahme in Tumorzellen vor allem von der Größe und der Ladung der Partikel abhängt. In orientierenden Studien wurde von den Erfindern beobachtet, daß die Verteilung im Tumorgewebe (z.B. des Mamma-Karzinoms) in den interstitiellen Raum des Gewebes (Mikrokapillaren, Septen, Lobuli) bei neutral bis negativ geladenen Teilchen sehr hoch ist. Unterstützt man die Verteilung derartiger Teilchen durch Wärme, so wird dieser Prozeß der Gleichverteilung noch wesentlich verstärkt. Es wird angenommen, daß neutrale bis negativ geladene Teilchen wenig Wechselwirkungen mit den extrazellulären Rezeptormolekülen und der Glycokalyx eingehen. Dies erklärt sich durch die viel häufiger negativ geladenen Ionenkanäle und integralen Proteine auf den Oberflächen der Zellen. Fast immer werden positiv geladene Ionen eingeschleust, die entweder der Signalweiterleitung (z.B. im Fall von Ca²⁺) oder der Erhaltung des osmotischen Gleichgewichts (z.B. Na⁺, K⁺) dienen. Die eher unspezifische Aufnahme erfolgt über positiv geladene Gruppen extrazellulärer Teilchen, weil die Zelle hier in den meisten Fällen verwertbare Biomoleküle einschleusen kann. Darüber hinaus werden auch biogene Zucker erkannt und eingeschleust, wobei diese Einschleusung bei Tumorzellen aufgrund entdifferenzierter Rezeptorproteine weniger spezifisch verläuft. Weiter muß die Tumorzelle aufgrund der höheren Stoffwechselaktivität und der häufig vorhandenen Sauerstoffunterversorgung der Tumorgewebe in viel höherem Maße eine anaerobe Glykolyse durchrühren als normale Zellen, was zum Teil auch eine Übersäuerung des Tumormilieus durch Anhäufung von Lactat zur Folge hat. Eine weitere Folge ist aber auch, daß die Tumorzellen durch die viel geringere Energieausbeute der anaeroben Stoffwechselwege viel mehr energiereiches Substrat verbrauchen, welches deshalb in großen Mengen in die Zellen eingeschleust werden muß. Da die Endozytose selbst ebenfalls ein energieverbrauchender Prozeß ist, besteht für die Tumorzelle ein Wettlauf mit der Zeit: durch höhere Einschleusungsraten wird zwar mehr Substrat eingeschleust, doch wird auch viel minderwertiges Material aufgenommen, das später kaum Energieausbeute bringt. Zudem wird durch die fortwährenden Teilungs- und Syntheseprozesse soviel Energie verbraucht, daß die üblicherweise streng kontrollierte Aufnahme von Stoffen aus der Umgebung nicht ausreichen und in diesem Fall ein großer Teil der Zellen absterben würde. Es ist daher einsichtig, daß die Tumorzelle mit einer hohen unspezifischen intrazellulären Aufnahme einen Überlebensvorteil hat, da sie aus der Zerlegung "grober" Bausteine mit Sicherheit schneller und mehr Energie gewinnen kann, als bei der hochselektiven Aufnahme weniger, spezifischer Grundbausteine.

Durch Beobachtungen in der Zellkultur und im Experimentaltumor wurde von den Erfindern festgestellt, daß die intrazelluläre Aufnahme (stark) positiv geladener Teilchen in Tumorzellen bis zu 1000 Mal höher ist als diejenige vergleichbarer Teilchen mit neutraler oder negativer Oberflächenladung. Dies wird auf die hohe Affinität der positiven Ladungen der Teilchen gegenüber den vielen negativ geladenen integralen Proteinen und Rezeptoren auf der Zelloberfläche zurückgeführt. Betrachtet man einen kürzeren Zeitraum, z.B. 6 bis 48 Stunden, so nehmen die Stoffwechsel- und Teilungs-aktiveren Tumorzellen selbst bei gleicher Affinität der Teilchen gegenüber der Oberfläche der normalen Zellen viel höhere Mengen derartiger Teilchen auf als normale Zellen. Wenn man zusätzlich die geringere Aufnahmespezifität der Tumorzellen berücksichtigt, so ergibt sich insgesamt ein beträchtlicher Unterschied der zellulären Aufnahme, die sich theoretisch therapeutisch nutzen lassen sollte. Hierfür ist keine systemische Anreicherung erforderlich, sondern lediglich die geschickte Nutzung von Oberflächenladungen zum Anheften der Teilchen an die Zelloberflächen der Tumorzellen.

Teilchen mit der höchsten erreichbaren positiven Außenladung werden elektrostatisch binnen weniger Sekunden an die Zellen gebunden und im Falle der Tumorzellen auch innerhalb von nur 2 bis 6 Stunden in solchen Mengen internalisiert, daß allein durch den intrazellulären Anteil der (Nano-)Teilchen kompakte Zellpellets in vitro durch ein äußeres magnetisches Wechselfeld aufgeheizt (auf 45-47°C) und inaktiviert werden können. In vivo findet man jedoch eine sehr schlechte Verteilung solcher (hoch) positiv geladenen Teilchen im Gewebe. Im Vergleich dazu haben neutrale oder negativ geladene Teilchen bessere Verteilungseigenschaften im Gewebe, werden aber weniger gut in die Zellen eingeschleust, sondern stattdessen vorwiegend vom RES abtransportiert. So ergaben z.B. Untersuchungen mit Dextran-beschichteten Magnetit-Teilchen, daß das Dextran endogen abgebaut wurde und dadurch eine optimale Leistungsaufnahme innerhalb der Tumorzellen verhindert wurde. Weiter wurde von den Erfindern gefunden, daß mit einem positive Ladungen aufweisenden Überzug (z.B. auf Aminosilan-Basis) versehene Magnetit-Teilchen zwar nicht endogen abgebaut wurden, sich aber auch schlecht im Gewebe verteilten. Es wäre deshalb wünschenswert, Teilchen zur Verfügung zu haben, die die Eigenschaften der beiden soeben beschriebenen (Magnetit-) Teilchen in sich vereinigen, nämlich zum einen eine gute Verteilung im Tumorgewebe zeigen und zum anderen auch gut von den Tumorzellen aufgenommen werden.

Erfindungsgemäß wurde nun gefunden, daß derartige Teilchen bereitgestellt werden können, indem man einen (vorzugsweise superparamagnetischen) Eisenoxid-haltigen Kern mit mindestens zwei Schalen (Hüllen) versieht, wobei die dem Kern benachbarte Schale viele positiv geladene funktionelle Gruppen aufweist, die eine leichte Aufnahme der so umhüllten Eisenoxid-haltigen Kerne in das Innere der Tumorzellen ermöglicht, wobei diese innere Schale auch so langsam vom (Tumor-)Gewebe abgebaut wird, daß die mit dieser Schale umgebenen Kerne ausreichend Zeit haben, um sich an die Zelloberfläche anzuheften (z.B. durch elektrostatische Wechselwirkungen zwischen den positiv geladenen Gruppen und negativ geladenen Gruppen auf der Zelloberfläche) und anschließend ins Zellinnere aufgenommen zu werden. Im Gegensatz dazu wird bzw. werden die äußere(n) Schale(n) von Spezies aufgebaut, die die darunterliegenden positiv geladenen Gruppen der inneren Schale abschirmen (maskieren) bzw. kompensieren oder sogar überkompensieren (z.B. durch vorhandene negativ geladene funktionelle Gruppen), so daß das die äußere(n) Schale(n) aufweisende nanoskalige Teilchen nach außen hin insgesamt neutral oder negativ geladen erscheint. Des weiteren wird bzw. werden die äußeren Schalen (deutlich) schneller als die innerste Schale vom Körpergewebe abgebaut, wobei diese Geschwindigkeit aber noch immer so gering ist, daß die Teilchen ausreichend Zeit haben, um sich im Gewebe zu verteilen, wenn sie (z.B. in Form eines magnetischen Fluids) punktförmig ins Gewebe injiziert werden. Im Zuge des Abbaus dieser äußere(n) Schale(n) wird die dem Kern benachbarte Schale mit der Zeit freigelegt. Als Ergebnis werden die umhüllten Kerne aufgrund der äußeren Schale(n) (und ihrer Elektroneutralität oder negativen Ladung nach außen hin) zunächst gut im Tumorgewebe verteilt und nach ihrer Verteilung aufgrund der durch den biologischen Abbau der äußeren Schale(n) freigelegten innersten Schale (und insbesondere deren positiv geladener Gruppen) auch gut in das Innere der Tumorzellen eingeschleust (bzw. zunächst an deren Oberfläche gebunden).

Gegenstand der vorliegenden Erfindung sind demgemäß nanoskalige Teilchen mit einem Eisenoxid-haltigen Kern (der ferro-, ferri- oder vorzugsweise superparamagnetisch ist) und mindestens zwei diesen Kern umgebenden Schalen, wobei es sich bei der dem Kern benachbarten (innersten) Schale um eine Hülle handelt, die über zur Bildung von kationischen Gruppen befähigte Gruppen verfügt und vom menschlichen oder tierischen Körpergewebe so langsam abgebaut wird, daß eine Assoziation des mit dieser Hülle umgebenen Kerns mit der Oberfläche von Zellen bzw. eine Aufnahme dieses Kerns ins Innere von Zellen möglich ist, und die äußere(n) Schale(n) von neutrale und/oder anionische Gruppen aufweisenden Spezies aufgebaut wird bzw. werden, die die nanoskaligen Teilchen nach außen hin neutral oder negativ geladen erscheinen lassen, und vom menschlichen oder tierischen Körpergewebe unter Freilegung der darunterliegenden Schale(n) schneller als die innerste Schale, aber noch ausreichend langsam abgebaut wird bzw. werden, um eine ausreichende Verteilung der nanoskaligen Teilchen in einem damit punktuell infiltrierten Körpergewebe zu gewährleisten.

Vorzugsweise handelt es sich bei der Kern-Substanz um ein reines Eisenoxid, insbesondere Magnetit und/oder Maghemit (γ-Fe₂O₃). Andere Kern-Substanzen, die erfindungsgemäß eingesetzt werden können, sind z.B. andere reine Eisenoxide, aber auch eisenhaltige Mischoxide, z.B. solche der allgemeinen Formel Me(II)Fe₂O₄, in der Me(II) vorzugsweise Zn, Cu, Co, Ni oder Mn repräsentiert (im Falle von Me(II)=Fe resultiert Magnetit). Weiter können auch andere als die obigen Metalle in den Kernen vorhanden sein, z.B. Erdalkalimetalle wie Ca und Mg. Ganz allgemein beträgt der Gehalt an von Eisenatomen verschiedenen Metallatomen in der Kern-Substanz vorzugsweise nicht mehr als 70, und inbesondere nicht mehr als 35 Metallatom-%. Bevorzugt sind die Eisenoxidteilchen jedoch reine Eisenoxidteilchen und insbesondere solche, die sowohl Fe(III) als auch Fe(II) enthalten, wobei das Verhältnis Fe(II)/Fe(III) vorzugsweise 1/1 bis 1/3 beträgt.

Unter "nanoskaligen Teilchen" werden in der vorliegenden Beschreibung und in den Ansprüchen Teilchen mit einer durchschnittlichen Teilchengröße (bzw. einem durchschnittlichen Teilchendurchmesser) von nicht mehr als 100 nm, vorzugsweise nicht mehr als 50 nm und insbesondere nicht mehr als 30 nm verstanden. Erfindungsgemäß weisen die nanoskaligen Teilchen vorzugsweise eine mittlere Teilchengröße im Bereich von 1 bis 40 nm, bevorzugter 3 bis 30 nm, auf, wobei eine Teilchengröße von nicht mehr als 30 nm normalerweise die Voraussetzung für Superparamagnetismus ist.

Besonders bevorzugt umfaßt der Kern der erfindungsgemäßen nanoskaligen Teilchen (superparamagnetischen) Magnetit, Maghemit oder stöchiometrische Zwischenformen derselben.

Die erfindungsgemäßen nanoskaligen Teilchen weisen in der Regel lediglich zwei Schalen auf. Es ist jedoch auch möglich, mehr als eine äußere Schale vorzusehen, z.B. zwei Schalen, die von unterschiedlichen Spezies aufgebaut werden.

Bei der freigelegten innersten (kernnächsten) Schale handelt es sich um eine Hülle, die vom menschlichen oder tierischen Körpergewebe (insbesondere Tumorgewebe) relativ langsam abgebaut wird und über kationische Gruppen bzw. über Gruppen, die zur Bildung von kationischen Gruppen in der Lage sind, verfügt. In der Regel handelt es sich bei diesen Gruppen um (positiv geladene) Aminogruppen, wenngleich auch andere positiv geladene bzw. aufladbare Gruppen erfindungsgemäß einsetzbar sind.

Die Kerne der erfindungsgemäßen nanoskaligen Teilchen können auf beliebige und dem Fachmann bekannte Art und Weise mit der innersten Schale versehen werden. Es muß lediglich gewährleistet sein, daß die innerste Schale im (Tumor)gewebe ausreichend langsam abgebaut wird, um eine Anhaftung der Teilchen an die Zelloberfläche und eine Einschleusung der Teilchen in die Zellen (vorzugsweise Tumorzellen) zu ermöglichen, und einige - vorzugsweise möglichst viele - kationische Gruppen aufweist. Normalerweise verfügt die innerste Schale im Durchschnitt über mindestens 50, vorzugsweise mindestens 100 und insbesondere mindestens 500 kationische Gruppen (z.B. positiv geladene Aminogruppen). Gemäß einer bevorzugten Ausführungsform der vorliegenden Erfindung wird die Hülle durch Einsatz von monomeren Aminosilanen wie z.B. 3-Aminopropyltriethoxysilan, 2-Aminoethyl-3-aminopropyltrimethoxysilan, Trimethoxysilylpropyldiethylentriamin und N-(6-Aminohexyl)-3-aminopropyltrimethoxysilan bereitgestellt. Vorzugsweise werden diese Aminosilane auf bekannte Art und Weise auf die Kerne aufgebracht und zur Erzielung einer hohen Stabilität polykondensiert. Ein hierfür geeignetes Verfahren ist z.B. in der DE-A-19614136 beschrieben. Auf die diesbezügliche Offenbarung wird hiermit ausdrücklich Bezug genommen. Ein weiteres Verfahren, das sich zur Bereitstellung einer kationische Gruppen aufweisenden innersten Schale um den Eisenoxid-haltigen Kern eignet, ist z.B. der DE-A-19515820 zu entnehmen. Selbstverständlich können auch andere Verfahren zu diesem Zweck eingesetzt werden.

Auf der beschriebenen innersten Schale werden erfmdungsgemäß eine oder mehrere (vorzugsweise eine) äußere Schalen vorgesehen. In der folgenden Beschreibung wird angenommen, daß lediglich eine äußere Schale vorliegt. Sollte mehr als eine äußere Schale gewünscht werden, wird mit den zusätzlichen Schalen analog verfahren.

Wie oben bereits erläutert, dient die äußere Schale dazu, eine gute Verteilung der mit der inneren Schale versehenen Eisenoxid-haltigen Kerne im Tumorgewebe zu erzielen, wobei sie, nachdem sie ihren Zweck erfüllt hat, biologisch (d.h. Gewebe-)abbaubar sein muß, um die darunterliegende innere Schale, die die leichte Aufnahme ins Innere der Zellen bzw. eine Assoziation mit der Oberfläche der Zellen ermöglicht, freizulegen. Die äußere Schale wird von Spezies aufgebaut, die keine positiv geladenen funktionellen Gruppen aufweisen, sondern im Gegenteil vorzugsweise über negativ geladene funktionelle Gruppen verfügen, so daß die nanoskaligen Teilchen nach außen insgesamt neutral (entweder durch Abschirmung (Maskierung) der positiven Ladungen im Inneren und/oder Neutralisation derselben durch negative Ladungen, wie sie beispielsweise von Carboxylgruppen bereitgestellt werden können) oder sogar negativ geladen erscheinen (z.B. durch überschüssige negativ geladene Gruppen). Erfindungsgemäß können zu diesem Zweck z.B. leicht (schnell) biologisch abbaubare Polymere mit für die Ankopplung an die darunterliegende Schale (insbesondere innerste Schale) geeigneten Gruppen eingesetzt werden, z.B. (Co)polymere auf der Basis von α-Hydroxycarbonsäuren (wie Polymilchsäure, Polyglycolsäure und Copolymere dieser Säuren), Polyolen (wie z.B. Polyethylenglykol) oder Polysäuren (z.B. Sebacinsäure). Besonders bevorzugt werden zu diesem Zweck gegebenenfalls modifizierte, natürlich vorkommende Substanzen, insbesondere Biopolymere. Unter den Biopolymeren sind beispielsweise die Kohlenhydrate (Zucker) und hier insbesondere die Dextrane zu nennen. Um in diesen neutralen Molekülen negativ geladene Gruppen zu erzeugen, kann man z.B. schwache Oxidationsmittel einsetzen, die einen Teil der Hydroxyl- bzw. Aldehydfunktionen in (negativ geladene) Carboxylgruppen umwandelt. Es muß jedoch betont werden, daß man beim Aufbau der äußeren Hülle nicht auf Kohlenhydrate oder die anderen oben angegebenen Spezies beschränkt ist, sondern daß vielmehr auch beliebige andere natürlich vorkommende oder synthetische Substanzen eingesetzt werden können, solange sie den Anforderungen hinsichtlich biologischer Abbaubarkeit (z.B. enzymatisch) und Ladung bzw. Ladungsmaskierung gerecht werden.

Die äußere Schicht kann auf dem Fachmann bekannte Art und Weise an die innere Schicht (bzw. eine darunterliegende Schicht) gekoppelt werden. Diese Kopplung kann z.B. elektrostatischer, kovalenter oder koordinativer Natur sein. Im Falle von kovalenten Wechselwirkungen können beispielsweise die üblichen Bindungs-bildenden Reaktionen der organischen Chemie eingesetzt werden, wie z.B. Esterbildung, Amidbildung und Iminbildung. Beispielsweise ist es möglich, einen Teil der oder alle Aminogruppen der innersten Schale mit Carboxylgruppen oder Aldehydgruppen entsprechender für den Aufbau der äußeren Schale(n) herangezogener Spezies umzusetzen, wodurch diese Aminogruppen unter Bildung von (Poly-)Amiden oder Iminen verbraucht (maskiert) werden. Der biologische Abbau der äußere(n) Schale(n) kann dann beispielsweise durch (z.B. enzymatische) Spaltung dieser Bindungen erfolgen, wodurch gleichzeitig die Aminogruppen regeneriert werden.

Obwohl die wesentlichen Elemente der erfindungsgemäßen nanoskaligen Teilchen (i) der Eisenoxid-haltige Kern, (ii) die im freigelegten Zustand positiv geladene, biologisch langsamer abbaubare innere Schale und (iii) die biologisch schneller abbaubare äußere Schale, die die nanoskaligen Teilchen nach außen hin insgesamt neutral oder negativ geladen erscheinen läßt, sind, können die erfindungsgemäßen Teilchen noch weitere, zusätzliche Komponenten aufweisen. In diesem Zusammenhang wären insbesondere Substanzen zu nennen, die mit Hilfe der erfindungsgemäßen Teilchen in das Innere von Zellen (vorzugsweise Tumorzellen) geschleust werden sollen, um dort die Wirkung der durch ein magnetisches Wechselfeld angeregten Kerne zu unterstützen oder eine davon unabhängige Funktion zu erfüllen. Derartige Substanzen werden (vor dem Aufbau der äußeren Schale(n)) vorzugsweise über kovalente Bindungen oder elektrostatische Wechselwirkungen an die innere Schale geknüpft. Dies kann nach denselben Mechanismen wie im Falle der Anbindung der äußeren Schale an die innere Schale erfolgen. So könnte z.B. im Falle der Verwendung von Aminosilanen als die innere Schale aufbauende Verbindungen ein Teil der vorhandenen Aminogruppen für die Anbindung derartiger Substanzen eingesetzt werden. Es müssen jedoch in diesem Fall (nach dem Abbau der äußeren Schale(n)) noch genügend Aminogruppen verbleiben, um die reibungslose Einschleusung der Eisenoxid-haltigen Kerne in das Zellinnere zu gewährleisten. In der Regel sollten nicht mehr als 10% der vorhandenen Aminogruppen für die Einschleusung anderer Substanzen in das Zellinnere verbraucht werden. Alternativ oder kumulativ ist es z.B. aber auch möglich, von Aminosilanen verschiedene Silane mit anderen funktionellen Gruppen zum Aufbau der inneren Schale einzusetzen, und diese anderen funktionellen Gruppen dann für die Anbindung anderer Substanzen und/oder der äußeren Schale an die innere Schale einzusetzen. Beispiele für andere funktionelle Gruppe sind z.B. ungesättigte Bindungen oder Epoxygruppen, wie sie beispielsweise durch mit (Meth)acrylgruppen oder Epoxygruppen versehene Silane bereitgestellt werden.

Erfindungsgemäß besonders bevorzugt ist es, an die innere Hülle Substanzen zu knüpfen, die erst bei leicht erhöhten Temperaturen, wie sie durch die Anregung der Eisenoxid-haltigen Kerne der erfindungsgemäßen Teilchen durch ein magnetisches Wechselfeld erzeugt werden, ihre volle Wirksamkeit entfalten, wie z.B. thermosensitive Chemotherapeutika (Cytostatika, Thermosensibilisatoren wie Doxorubicin, Proteine usw.).

Koppelt man z.B. einen Thermosensibilisator an die innere Schale (z.B. über Aminogruppen), werden die entsprechenden Thermosensibilisator-Moleküle erst nach Abbau der äußeren (z.B. Dextran-) Hülle unter Wärmeentwicklung (durch das magnetische Wechselfeld) reaktiv. Auf diese Weise kann eine konstante, biogen nicht beeinflußbare Leistungsabsorption im Tumorgewebe erzeugt werden, die durch die Wirkung des Thermosensibilisators (cytotoxische Wirkung bei Doxorubicin z.B. erst ab ca. 43°C) noch verstärkt wird.

Zur Erzielung optimaler Ergebnisse, z.B. bei der Tumortherapie, muß die Erregerfrequenz des Magnetwechselfeld-Applikators zwecks Erzielung einer maximalen Energieausbeute auf die Größe der Kerne der erfindungsgemäßen nanoskaligen Teilchen abgestimmt werden. Durch die gute Verteilung der Teilchensuspension im Tumorgewebe können benachbarte, nur wenige Mikrometer lange Zwischenräume einerseits durch die Wärmeentwicklung und andererseits durch die Wirkung des Thermosensibilisators vor allem bei mehrmaliger Anregung durch das Wechselfeld in einem aus der Gentherapie bekannten sogenannten "Bystander"-Effekt überbrückt werden, so daß letztlich das gesamte Tumorgewebe zerstört wird.

Teilchen, die das Tumorgewebe verlassen, werden von Kapillaren und dem Lymphsystem in die Blutbahn und von dort aus in Leber und Milz transportiert. In diesen Organen findet dann der biogene Abbau der Teilchen bis hin zu den Kernen (in der Regel Eisenoxide bzw. Eisenionen) statt, die einerseits ausgeschieden und andererseits auch resorbiert und in den Eisenpool des Körpers eingeschleust werden. Liegen also zwischen der intraläsionalen Magnetflüssigkeitsapplikation und der Wechselfelderregung wenigstens 0,5 - 2 Stunden, hat sich die Umgebung des Tumors selbst von den Magnetteilchen "gereinigt", so daß während der Wechselfeldanregung tatsächlich nur die Läsion, nicht aber die Umgebung, erwärmt wird.

Im Gegensatz zu hochmolekularen Substanzen verlassen Nanopartikel das Gewebe, in das sie injiziert wurden, nicht, sondern bleiben in Gewebezwischenräumen hängen. Lediglich über während der Applikation perforierte Gefäße findet ein Abtransport statt. Hochmolekulare Substanzen verlassen das Gewebe dagegen schon durch Diffusion und Tumordruck oder werden durch Bioabbau inaktiviert. Diese Prozesse sind mit den erfindungsgemäßen nanoskaligen Teilchen nicht möglich, da diese einerseits schon so klein sind, daß sie in Gewebezwischenräume eindringen können (was mit Teilchen im µm-Bereich, z.B. Liposomen, nicht möglich ist), jedoch größer sind als Moleküle und dadurch das Gewebe nicht über Diffusion und Kapillardruck verlassen können. Darüber hinaus sind die nanoskaligen Teilchen osmotisch nicht aktiv und beeinflussen ohne Anwendung eines magnetischen Wechselfeldes das Tumorwachstum kaum, was für eine optimale Verteilung der Teilchen in Tumorgewebe unbedingt erforderlich ist.

Findet eine frühzeitige Beladung des Primärtumors statt, so werden die Teilchen in hohem Maße von den Tumorzellen aufgenommen und später auch mit einer Wahrscheinlichkeit von 50% über das elterliche Cytoplasma an die Tochterzellen weitergegeben. Setzt man deshalb auch die weitere Umgebung des Tumors bzw. bekannte Orte einer Metastasierung ebenfalls dem magnetischen Wechselfeld aus, so werden einzelne, auch weit vom Primärtumor entfernte Tumorzellen von der Behandlung erfaßt. Vor allem die Therapie von befallenen Lymphknoten kann so selektiver als bei der Chemotherapie angegangen werden. Zusätzliche Einwirkungen von statischen Magnetfeldgradienten an Risikoorten einer späteren Wechselfeldanwendung können die Trefferquote beladener Tumorzellen noch erhöhen.

Wie bereits oben erwähnt, werden an die erfindungsgemäßen nanoskaligen Teilchen (konkret deren innere Schale) vorzugsweise Thermosensibilisatoren und/oder Cytostatika gebunden. Erst durch die magnetische Kopplung der magnetischen Teilchen mit dem magnetischen Wechselfeld und der daraus resultierenden Wärmeentwicklung kommt es zur Aktivierung oder auch Freisetzung von cytotoxisch wirkenden Substanzen, die (vorzugsweise) an die erfindungsgemäßen Teilchen gekoppelt worden sind.

Durch die zweistufige intraläsionale Applikation ist keine selektive Anreicherung notwendig. Vielmehr reicht die in der Routinediagnose festgestellte exakte Lokalisation der Läsion mit nachfolgender stereotaktisch oder mit Navigationssystemen (Robotik) geführter Infiltration der Magnetflüssigkeit in eine beliebig kleine (oder größere) Zielregion aus.

Durch Kombination mit dem statischen Magnetfeldgradienten wird eine regional selektive Chemoembolisation ermöglicht, da durch Wärme nicht nur das vorzugsweise an den erfindungsgemäßen Teilchen befindliche cytostatische Agens aktiviert wird, sondern durch das statische Feld eine reversible Aggregation der Teilchen und somit eine gezielte Embolisation hervorgerufen werden kann.

Neben der Tumortherapie liegen weitere Anwendungen der erfindungsgemäßen nanoskaligen Teilchen (gegebenenfalls ohne die äußere(n) Schale(n)) in der wärmeinduzierten Lysis von Mikrokapillarverschlüssen (Thromben) beliebiger Lokalisation in operativ nicht zugänglichen Regionen und der sukzessiven Auflösung von Thromben an Coronargefäßen. Zu diesem Zweck können an die innere Schale der erfindungsgemäßen Teilchen z.B. proteolytische Enzyme gekoppelt werden, die unter Einwirkung von Wärme bis zum 10-fachen ihrer Aktivität enfalten bzw. durch Erwärmung erst reaktiv werden. Nach intraarterieller Punktion des Gefäßes in unmittelbarer Nähe des Verschlusses werden die Teilchen automatisch zum "Staupunkt" transportiert (z.B. unter MRT-Kontrolle). Angiographisch wird eine faseroptische Temperatursonde mit einem Durchmesser von beispielsweise 0,5 mm eingebracht und in der Nähe des Stauortes wird die Temperatur gemessen, während wieder durch äußeres Anlegen eines magnetischen Wechselfeldes eine mikroregionale Aufheizung und Aktivierung der proteolytischen Enzyme veranlaßt wird. Im Prinzip kann bei präziser Applikation der Magnetflüssigkeit und MRT-Kontrolle auch auf die Temperaturmessung verzichtet werden, da die zu erwartende Leistungsabsorption bereits aus der applizierten Magnetflüssigkeitsmenge unter der bekannten Feldstärke und Frequenz relativ genau abgeschätzt werden kann. In Intervallen von etwa 6 bis 8 Stunden wird das Feld erneut angelegt. In der erregungsfreien Zeit hat der Körper die Möglichkeit, Zelltrümmer zum Teil abzutransportieren, bis schließlich, vom Körper selbst unterstützt, der Verschluß aufgehoben ist. Die Durchwanderung der erfindungsgemäßen Teilchen durch die Herzkammern und Gefäße ist bedingt durch deren geringe Größe unkritisch. Schließlich gelangen die Teilchen via RES zwecks Bioabbau wieder in Leber und Milz.

Neben der klassischen Hyperthermie bei Temperaturen bis 46/47°C kann mit den erfindungsgemäßen nanoskaligen Teilchen auch eine Thermoablation durchgeführt werden. Nach dem Stand der Technik verwendet man für thermoablative Zwecke hauptsächlich interstitielle Lasersysteme, die zum Teil auch in der Chirurgie eingesetzt werden. Ein großer Nachteil dieses Verfahrens ist die hohe Invasivität der mikrokathetergeführten faseroptischen Laserzuführung und die schwierige Kontrolle der Zielvolumenausdehnung. Für solche Zwecke können weniger traumatisch als bei der Lasertechnik die erfindungsgemäßen Nanoteilchen verwendet werden: nach MRT-gestützter Anreicherung der Partikelsuspension in der Zielregion können bei höheren Wechselfeldamplituden auch Temperaturen über 50°C homogen erzeugt werden. Die Temperaturkontrolle kann z.B. ebenfalls über eine hauchdünne faseroptische Sonde von weniger als 0,5 mm Durchmesser erfolgen. Die Energieabsorption selbst ist nicht-invasiv.

## Patentansprüche

1. Nanoskalige Teilchen mit einem Eisenoxid-haltigen Kern und mindestens zwei diesen Kern umgebenden Schalen, wobei es sich bei der dem Kern benachbarten (innersten) Schale um eine Hülle handelt, die über zur Bildung von kationischen Gruppen befähigte Gruppen verfügt und vom menschlichen oder tierischen Körpergewebe so langsam abgebaut wird, daß nach Freilegung der innersten Schale durch Abbau der äußeren Schale(n) eine Assoziation des mit dieser Hülle umgebenen Kerns mit der Oberfläche von Zellen bzw. eine Aufnahme dieses Kerns ins Innere von Zellen möglich ist, und die äußere(n) Schale(n) von neutrale und/oder anionische Gruppen aufweisenden Spezies aufgebaut wird bzw. werden, die die nanoskaligen Teilchen nach außen hin neutral oder negativ geladen erscheinen lassen, und vom menschlichen oder tierischen Körpergewebe unter Freilegung der darunterliegenden Schale(n) schneller als die innerste Schale, aber noch ausreichend langsam abgebaut wird bzw. werden, um eine Verteilung der nanoskaligen Teilchen in einem damit punktuell infiltrierten Körpergewebe zu gewährleisten.

2. Nanoskalige Teilchen nach Anspruch 1, **dadurch gekennzeichnet, daß** der Kern Magnetit, Maghemit oder stöchiometrische Zwischenformen derselben umfaßt.

3. Nanoskalige Teilchen nach irgendeinem der Ansprüche 1 und 2, **dadurch gekennzeichnet, daß** die Kerne eine mittlere Teilchengröße von 1 bis 40 nm, vorzugsweise 3 bis 30 nm, aufweisen.

4. Nanoskalige Teilchen nach irgendeinem der Ansprüche 1 bis 3, **dadurch gekennzeichnet, daß** die Kerne superparamagnetisch sind.

5. Nanoskalige Teilchen nach irgendeinem der Ansprüche 1 bis 4, **dadurch gekennzeichnet, daß** die Kerne von zwei Schalen umgeben sind.

6. Nanoskalige Teilchen nach irgendeinem der Ansprüche 1 bis 5, **dadurch gekennzeichnet, daß** die innerste Schale Aminogruppen aufweist.

7. Nanoskalige Teilchen nach irgendeinem der Ansprüche 1 bis 6, **dadurch gekennzeichnet, daß** die innerste Schale von (hydrolytisch) polykondensierbaren Silanen, insbesondere Aminosilanen, abgeleitet ist.

8. Nanoskalige Teilchen nach irgendeinem der Ansprüche 1 bis 7, **dadurch gekennzeichnet, daß** die innerste Schale durchschnittlich mindestens 50, vorzugsweise mindestens 100, positiv geladene Gruppen aufweist.

9. Nanoskalige Teilchen nach irgendeinem der Ansprüche 1 bis 8, **dadurch gekennzeichnet, daß** die äußere(n) Schale(n) gegebenenfalls modifizierte, natürlich vorkommende Substanzen, insbesondere Biopolymere, umfaßt bzw. umfassen.

10. Nanoskalige Teilchen nach irgendeinem der Ansprüche 1 bis 9, **dadurch gekennzeichnet, daß** die äußere(n) Schale(n) gegebenenfalls modifizierte Kohlenhydrate, insbesondere Dextrane, umfaßt bzw. umfassen.

11. Nanoskalige Teilchen nach Anspruch 10, **dadurch gekennzeichnet, daß** die Kohlenhydrate durch Carboxylgruppen modifiziert sind.

12. Nanoskalige Teilchen nach irgendeinem der Ansprüche 1 bis 11, **dadurch gekennzeichnet, daß** an die innerste Schale eine oder mehrere pharmakologisch aktive Spezies, vorzugsweise aus der Gruppe der Thermosensibilisatoren und thermosensitiven Chemotherapeutika, gebunden sind.

13. Magnetische Fluide zur Einbringung in den menschlichen oder tierischen Körper, enthaltend nanoskalige Teilchen nach irgendeinem der Ansprüche 1 bis 12.

14. Verwendung eines magnetischen Fluids nach Anspruch 13 zur Herstellung eines Arzneimittels für eine Tumortherapie durch Hyperthermie.

15. Verwendung eines magnetischen Fluids nach Anspruch 13 zur Herstellung eines Arzneimittels für eine wärmeinduzierte Lysis von Mikrokapillarverschlüssen (Thromben).

16. Verwendung eines magnetischen Fluids nach Anspruch 13 zur Herstellung eines Arzneimittels für eine Thermoablation.

17. Verwendung von nanoskaligen Teilchen nach irgendeinem der Ansprüche 1 bis 12 zur Herstellung eines Arzneimittels für eine Magnetwechselfeld-Behandlung bei homogener Verteilung und/oder lokaler/regionaler Konzentrationserhöhung und/oder Verminderung des "washout" in biologischen Geweben der nanoskaligen Teilchen oder von an diese Teilchen gekoppelten Spezies.

18. Verwendung nach Anspruch 17, **dadurch gekennzeichnet, daß** an die nanoskaligen Teilchen als Spezies Chemotherapeutika oder Isotopen gekoppelt sind.

## Claims

1. Nanoscale particles having an iron oxide-containing core and at least two shells surrounding said core, the (innermost) shell adjacent to the core being a coat that features groups capable of forming cationic groups and that is degraded by the human or animal body tissue at such a low rate that, after exposure of the innermost shell, an association of the core surrounded by said coat with the surface of cells and the incorporation of said core into the inside of cells, respectively, is possible, and the outer shell(s) being constituted by species having neutral and/or anionic groups which, from without, make the nanoscale particles appear neutral or negatively charged and which is (are) degraded by the human or animal body tissue to expose the underlying shell(s) at a rate which is higher than that for the innermost shell but still low enough to ensure a sufficient distribution of said nanoscale particles within a body tissue which has been punctually infiltrated therewith.

2. Nanoscale particles according to claim 1, wherein the core comprises magnetite, maghemite or stoichiometric intermediate forms thereof.

3. Nanoscale particles according to any one of claims 1 and 2, wherein the cores have an average particle size of 1 to 40 nm, preferably 3 to 30 nm.

4. Nanoscale particles according to any one of claims 1 to 3, wherein the cores are superparamagnetic.

5. Nanoscale particles according to any one of claims 1 to 4, wherein said cores are surrounded by two shells.

6. Nanoscale particles according to any one of claims 1 to 5, wherein the innermost shell features amino groups.

7. Nanoscale particles according to any one of claims 1 to 6, wherein the innermost shell is derived from (hydrolytically) polycondensable silanes, particularly aminosilanes.

8. Nanoscale particles according to any one of claims 1 to 7, wherein the innermost shell on average has at least 50, preferably at least 100, positively charged groups.

9. Nanoscale particles according to any one of claims 1 to 8, wherein the outer shell(s) comprise(s) optionally modified, naturally occurring substances, particularly biopolymers.

10. Nanoscale particles according to any one of claims 1 to 9, wherein the outer shell(s) comprise(s) optionally modified carbohydrates, particularly dextrans.

11. Nanoscale particles according to claim 10, wherein said carbohydrates are modified by carboxylic groups.

12. Nanoscale particles according to any one of claims 1 to 11, wherein one or more pharmacologically active species, preferably selected from the group consisting of thermosensitizers and thermosensitive chemotherapeutic agents, are linked to the innermost shell.

13. Magnetic fluids for introduction into the human or animal body, containing nanoscale particles according to any one of claims 1 to 12.

14. Use of a magnetic fluid according to claim 13 for the manufacture of a medicament for use in tumor therapy by hyperthermia.

15. Use of a magnetic fluid according to claim 13 for the manufacture of a medicament for use in the heat-induced lysis of clogged microcapillaries (thrombi).

16. Use of a magnetic fluid according to claim 13 for the manufacture of a medicament for use in thermoablation.

17. Use of nanoscale particles according to any one of claims 1 to 12 for the manufacture of a medicament for an alternating magnetic field treatment at homogeneous distribution and/or locally/regionally increased concentration and/or reduced washout in biological tissues of said nanoscale particles or species coupled to said nanoscale particles.

18. Use according to claim 17, wherein said species coupled to the nanoscale particles are selected from the group consisting of chemotherapeutical agents and isotopes.

## Revendications

1. Nanoparticules avec un noyau contenant de l'oxyde de fer et au moins deux enveloppes entourant ce noyau, l'enveloppe (interne) adjacente au noyau étant une couche comportant des groupes capables de former des groupes cationiques et qui est dégradée si lentement par les tissus du corps humain ou du corps animal que, après mise à nu de l'enveloppe interne suite à la décomposition de la ou des enveloppe(s) externe(s), le noyau entouré de cette enveloppe peut s'associer à la surface de cellules ou peut être absorbé par des cellules, et la ou les enveloppe(s) externe(s) sont constituée(s) d'espèces non-ioniques et/ou anioniques, qui confèrent aux nanoparticules une charge de surface nulle ou négative, et qui sont décomposées par les tissus du corps humain ou animal - libérant ainsi la ou les enveloppes sous-jacente(s) - plus vite que l'enveloppe la plus interne mais assez lentement pour garantir une répartition des nanoparticules dans un tissu corporel infiltré en un point donné par ces particules.

2. Nanoparticules selon la revendication 1, **caractérisées par le fait que** le noyau contient de la magnétite, de la maghémite ou des formes stoechiométriquement intermédiaires de celles-ci.

3. Nanoparticules selon l'une quelconque des revendications 1 et 2, **caractérisées par le fait que** les noyaux ont une taille moyenne comprise entre 1 et 40 nm, de préférence entre 3 et 30 nm.

4. Nanoparticules selon l'une quelconque des revendications 1 à 3, **caractérisées par le fait que** les noyaux sont constitués d'un matériau superparamagnétique.

5. Nanoparticules selon l'une quelconque des revendications 1 à 4, **caractérisées par le fait que** les noyaux sont entourés de deux enveloppes.

6. Nanoparticules selon l'une quelconque des revendications 1 à 5, **caractérisées par le fait que** l'enveloppe la plus interne contient des groupes amine.

7. Nanoparticules selon l'une quelconque des revendications 1 à 6, **caractérisées par le fait que** l'enveloppe la plus interne est dérivée de silanes, en particulier d'aminosilanes, susceptibles d'être polycondensés (par voie hydrolytique).

8. Nanoparticules selon l'une quelconque des revendications 1 à 7, **caractérisées par le fait que** l'enveloppe la plus interne contient en moyenne au moins 50, de préférence au moins 100 groupes portant une charge positive.

9. Nanoparticules selon l'une quelconque des revendications 1 à 8, **caractérisées par le fait que** la ou les enveloppe(s) externe(s) contiennent des substances naturelles, en particulier des biopolymères, éventuellement modifiées.

10. Nanoparticules selon l'une quelconque des revendications 1 à 9, **caractérisées par le fait que** la ou les couche(s) extérieure(s) contiennent des hydrates de carbone, en particulier des dextranes.

11. Nanoparticules selon la revendication 10, **caractérisées par le fait que** les hydrates de carbone sont modifiés par des groupes carboxyle.

12. Nanoparticules selon l'une quelconque des revendications 1 à 11, **caractérisées par le fait que** l'enveloppe la plus interne contient une ou plusieurs espèces à activité pharmacologique, de préférence choisies dans le groupe formé par les agents thermosensibilisateurs et les agents chimiothérapeutiques thermosensibles.

13. Fluide magnétique destiné à être introduit dans le corps humain ou animal, contenant des nanoparticules selon l'une quelconque des revendications 1 à 12.

14. Utilisation d'un fluide magnétique selon la revendication 13 pour la fabrication d'un médicament destiné à la thérapie de tumeurs par hyperthermie.

15. Utilisation d'un fluide magnétique selon la revendication 13, pour la préparation d'un médicament pour la lyse thermoinduite de bouchons microcapillaires (thrombus).

16. Utilisation d'un fluide magnétique selon la revendication 13, pour la fabrication d'un médicament destiné à une thermoablation.

17. Utilisation de nanoparticules selon l'une quelconque des revendications 1 à 12, pour la fabrication d'un médicament destiné au traitement par un champ magnétique alternatif avec répartition homogène et/ou concentration locale/régionale et/ou diminution du "washout" des nanoparticules ou de substances couplées à ces particules, dans des tissus biologiques.

18. Utilisation selon la revendication 17, **caractérisée par le fait que** des composés chimiothérapeutiques ou des isotopes sont couplés aux nanoparticules.
